# EUROPEAN PATENT SPECIFICATION

(11) **EP 3 797 155 B1**
(45) Date of publication and mention of the grant of the patent: **15.06.2022**
(21) Application number: 19730234.2
(22) Date of filing: 21.05.2019
(51) Int. Cl.: C12N 5/0783

(54) **OPTIMIZATION OF NK-92 CELL GROWTH USING POLOXAMER**
OPTIMIERUNG VON NK-92-ZELL-WACHSTUM UNTER VERWENDUNG VON POLOXAMER
OPTIMISATION DE LA CROISSANCE DE CELLULES NK-92 À L'AIDE DE POLOXAMÈRE

(30) Priority: 22.05.2018 US 201862674723 P
(43) Date of publication of application: 31.03.2021
(73) Proprietor: ImmunityBio, Inc., San Diego CA 92121 (US)
(72) Inventor: BESSETTE, Shannyn, San Diego, California 92121 (US); NGUYEN, Diemchi, San Diego, California 92121 (US); ALI, Syed Raza, San Diego, California 92121 (US); SAXENA, Manju, San Diego, California 92121 (US)
(74) Representative: Elkington and Fife LLP
(86) International application number: PCT/US2019/033312
(87) International publication number: WO 2019/226649

(56) References cited:
- WO-A1-98/08934
- US-A1- 2005 153 419
- TAM Y K ET AL: "Ex vivo expansion of the highly cytotoxic human natural killer cell-line NK-92 under current good manufacturing practice conditions for clinical adoptive cellular immunotherapy", CYTOTHERAPY, ISIS MEDICAL MEDIA, OXFORD, GB, vol. 5, no. 3, 1 January 2003 (2003-01-01) , pages 259-272, XP003027016, ISSN: 1465-3249, DOI: 10.1080/14653240310001523
- ANNE GIGOUT ET AL: "Chondrocytes Cultured in Stirred Suspension with Serum-Free Medium Containing Pluronic-68 Aggregate and Proliferate While Maintaining Their Differentiated Phenotype", TISSUE ENGINEERING PART A, vol. 15, no. 8, 1 August 2009 (2009-08-01) , pages 2237-2248, XP55618318, US ISSN: 1937-3341, DOI: 10.1089/ten.tea.2008.0256
- CURT I. CIVIN ET AL: "Automated leukocyte processing by microfluidic deterministic lateral displacement : Automated Microfluidic Blood Leukocyte Processing", NIH PUBLIC ACCESS AUTHOR MANUSCRIPT, vol. 89, no. 12, 22 November 2016 (2016-11-22), pages 1073-1083, XP055619089, ISSN: 1552-4922, DOI: 10.1002/cyto.a.23019
- T. THARMALINGAM ET AL: "Pluronic Enhances the Robustness and Reduces the Cell Attachment of Mammalian Cells", MOLECULAR BIOTECHNOLOGY, vol. 39, no. 2, 8 March 2008 (2008-03-08), pages 167-177, XP055129642, ISSN: 1073-6085, DOI: 10.1007/s12033-008-9045-8

## Description

### RELATED APPLICATION

This application claims priority to U.S. Provisional Application No. 62/674,723, filed on May 22, 2018.

### BACKGROUND

Natural killer (NK) cells are cytotoxic lymphocytes that constitute a major component of the innate immune system. NK cells, generally representing about 10-15% of circulating lymphocytes, bind and kill targeted cells, including virus-infected cells and many malignant cells, non-specifically with regard to antigen and without prior immune sensitization. Herberman et al., Science 214:24 (1981). Killing of targeted cells occurs by inducing cell lysis. NK cells used for this purpose are isolated from the peripheral blood lymphocyte ("PBL") fraction of blood from the subject, expanded in cell culture in order to obtain sufficient numbers of cells, and then re-infused into the subject. NK cells have been shown to be somewhat effective in both ex vivo therapy and in vivo treatment. However, such therapy is complicated by the fact that not all NK cells are cytolytic and the therapy is specific to the treated patient.

NK-92^{®} cells have previously been evaluated as a therapeutic agent in the treatment of certain cancers. Unlike NK cells, NK-92^{®} is a cytolytic cancer cell line, which was discovered in the blood of a subject suffering from a non-Hodgkins lymphoma and then immortalized *ex vivo.* NK-92^{®} cells lack the major inhibitory receptors that are displayed by normal NK cells, but retain the majority of the activating receptors. NK-92^{®} cells do not, however, attack normal cells nor do they elicit an unacceptable immune rejection response in humans. Characterization of the NK-92^{®} cell line is disclosed, e.g., in WO 1998/49268 and U.S. Patent No. 8,034,332. Tam et al (Cytotherapy 2003;5(3):259-72) describes a protocol that allows for >200-fold expansion of NK-92^{®} cells within a 2-2.5 week period under GMP standards, in quality and quantity suitable for clinical adoptive immunotherapy.

Although NK-92^{®} cells have tremendous therapeutic potential, growing NK-92^{®} cells in large scale has been a challenge, which limits the therapeutic applications of these cells. In particular, expansion is often accompanied with significant increases in culture precipitates and flocculants, a phenomenon commonly referred to as "clumping." These large sized precipitates cause many undesired consequences, including slow cell growth, low cell viability, and inaccurate cell counting, which may result in incorrect dosing formulation. Clumping in cell culture may also result in disturbance of cell bed in centrifugation, which leads to poor cell recovery during cell harvest, and inconsistent cell cytotoxicity against tumor target cells.

### BRIEF SUMMARY

Provided herein are methods of culturing NK-92^{®} cells using a growth media containing a non-ionic surfactant, wherein the NK-92^{®} cells have reduced clumping as compared to control NK-92^{®} cells that have been cultured in a control medium lacking the non-ionic surfactant. The growth medium comprises 0.025 to 0.9% of a non-ionic surfactant, e.g., Poloxamer 188. The NK-92^{®} cells may be modified to express one or more transgenes, for example, the NK-92^{®} cells can be modified to express a cytokine, a Fc receptor, a chimeric antigen receptor, or a combination thereof. Also provided herein are NK-92^{®} cell cultures comprising NK-92^{®} cells and a culture medium comprising 0.025%-0.9% of the non-ionic surfactant.

Provided herein is a method of culturing NK-92^{®} cells comprising culturing the NK-92^{®} cells in a culture medium comprising 0.025% to 0.9% of a non-ionic surfactant, wherein the NK-92^{®} cell culture has reduced clumping as compared to control NK-92^{®} cells that have been cultured in a control medium lacking the non-ionic surfactant. Optionally, the NK-92^{®} cells maintained substantially the same cytotoxicity as the control NK-92^{®} cells. Additionally, the cell culture is substantially free from clumping with the non-ionic surfactant is Poloxamer 188. Optionally, the cells are cultured in at least 2 liters of culture medium before visual benefits of the additive can be observed to the naked eye.

The culture medium for culturing NK-92^{®} cells may comprise from 0.025% to 0.06% of Poloxamer 188, e.g., 0.05% Poloxamer 188. Optionally the NK-92^{®} cell culture has reduced cell aggregates as compared to a control culture. In some cases, the reduction of the percentage of cell aggregates is at least 40%. Optionally, the NK-92^{®} cell culture has less than 6% cell aggregates after 3 days of culturing.

The NK-92^{®} cells cultured using the methods disclosed herein may have a viability of at least 80%. They may maintain the substantially the same cytotoxicity as the NK-92^{®} cells in the control culture. The NK-92^{®} cells may comprise a cytokine, Fc Receptor, chimeric antigen receptor or a combination thereof.

Also provided herein is a method of reducing fluocculants in a culture medium, the method comprising adding to the culture medium 0.025% to 0.9% of a non-ionic surfactant, wherein the culture medium has reduced fluocculants as compared to control culture lacking the non-ionic surfactant.

Also provided herien is a cell culture comprising NK-92^{®} cells and a culture medium comprising 0.025% to 0.9% of the non-ionic surfactant, wherein the cell culture has reduced clumping as compared to control culture comprising NK-92^{®} cells and a medium lacking the non-ionic surfactant. In some cases, non-ionic surfactant is Poloxamer 188. Optionally, the NK-92^{®} cells have been cultured for at least 3 days. Optionally, the cell culture is substantially free from clumping. Optionally, the NK-92^{®} cells maintain the substantially the same cytotoxicity as the NK-92^{®} cells in a control culture. Optionally, the cell culture of claim 14, wherein the cell culture comprises 0.025% to 0.06%, e.g., 0.05% of the Poloxamer 188. The NK-92^{®} cells may comprise a cytokine, Fc Receptor, chimeric antigen receptor, or a combination thereof. Optionally, the cell culture has a volume of at least 2 liters, e.g., at least 10 liters.

The foregoing general description and the following detailed description are exemplary and explanatory and are intended to provide further explanation of the disclosure. Other objects, advantages and novel features will be readily apparent to those skilled in the art.

### BRIEF DESCRIPTION OF THE DRAWINGS

The objects, features and advantages will be more readily appreciated upon reference to the following disclosure when considered in conjunction with the accompanying drawings.
FIG. 1 shows a first schematic plan of expanding haNK^{®} cells in a WAVE Bioreactor in the absence of Poloxamer 188 (Pluronic F-68). Clumping and low cell viability were observed.
FIG. 2 shows a second schematic plan of expanding haNK^{®} cells in a WAVE Bioreactor in the absence of Poloxamer 188 (Pluronic F-68) and results. Clumping and low cell viability were observed.
FIG. 3 shows a schematic plan of expanding haNK^{®} cells in a WAVE Bioreactor in the presence of 0.05% Poloxamer 188 (Pluronic F-68). The culture was free from clumping.
FIG. 4 shows a first schematic plan of expanding aNK^{™} cells in a WAVE Bioreactor in the absence of Poloxamer 188 (Pluronic F-68). Clumping was observed.
FIG. 5 shows a second schematic plan of expanding aNK^{™} cells in a WAVE Bioreactor in the absence of Poloxamer 188 (Pluronic F-68). Clumping was observed.
FIG. 6 shows a schematic plan of expanding aNK^{™} cells in a WAVE Bioreactor in the presence of 0.05% Poloxamer 188 (Pluronic F-68). The culture was free from clumping.
FIG. 7 shows a schematic plan of expanding haNK^{®} cells in WAVE Bioreactor in the absence or presence of various titrations of Poloxamer 188 (Pluronic F-68). Clumping in the haNK^{®} cultures was reduced as the concentration of Poloxamer 188 (Pluronic F-68) increased.
FIGs. 8A and 8B show the NC-200 profiles of haNK^{®} cells from a WAVE Bioreactor in the absence of Poloxamer 188 (Pluronic F-68) (FIG. 8A) and the presence of 0.05% Poloxamer 188 (Pluronic F-68) (FIG. 8B).
FIGs. 9A and 9B show the NC-200 profiles of aNK^{™} cells from a WAVE Bioreactor in the absence of Poloxamer 188 (Pluronic F-68) (FIG. 9A) and the presence of 0.05% Poloxamer 188 (Pluronic F-68) (FIG. 9B).
FIG. 10 shows a picture of NK-92^{®} cells grown in the WAVE bags in the absence of Poloxamer 188, where clumping was observed.

### DETAILED DESCRIPTION

Provided herein are methods of culturing NK-92^{®} cells using growth media that can reduce clumping. In some cases, the NK-92^{®} cells are cultured in a growth media and the cells are substantially free of cell lumping. The growth medium comprises 0.025 to 0.9% of a non-ionic surfactant, e.g., Poloxamer 188. The NK-92^{®} cells may be modified to express one or more transgenes, for example, a cytokine, a Fc receptor, a chimeric antigen receptor, or a combination thereof. Also provided herein are NK-92^{®} cell cultures comprising NK-92^{®} cells and a culture medium comprising 0.025%-0.9% of the non-ionic surfactant.

### TERMINOLOGY

Unless defined otherwise, all technical and scientific terms used herein have the same meaning as commonly understood by one of ordinary skill in the art.

In this specification and in the claims that follow, reference will be made to a number of terms that shall be defined to have the following meanings:

The terminology used herein is for the purpose of describing particular embodiments only and is not intended to be limiting. As used herein, the singular forms "a," "an" and "the" are intended to include the plural forms as well, unless the context clearly indicates otherwise. Thus, for example, reference to "a natural killer cell" includes a plurality of natural killer cells.

All numerical designations, e.g., pH, temperature, time, concentration, amounts, and molecular weight, including ranges, are approximations which are varied (+) or (-) by increments of 0.1 or 1.0, where appropriate. It is to be understood, although not always explicitly stated, that all numerical designations may be preceded by the term "about." All concentrations in this disclosure are volume/volume concentrations.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

It is also to be understood, although not always explicitly stated, that the reagents described herein are merely exemplary and that equivalents of such are known in the art.

"Optional" or "optionally" means that the subsequently described event or circumstance can or cannot occur, and that the description includes instances where the event or circumstance occurs and instances where it does not.

For purposes of this invention and unless indicated otherwise, the term "NK-92^{®}" or "NK92^{®}" is intended to refer to the original NK-92^{®} cell lines as well as NK-92^{®} cell lines, clones of NK-92^{®} cells, and NK-92^{®} cells that have been modified (e.g., by introduction of exogenous genes). NK-92^{®} cells and exemplary and non-limiting modifications thereof are described in U.S. Patent Nos. 7,618,817; 8,034,332; 8,313,943; 9,181,322; 9,150,636; and published U.S. Application No. 10/008,955, and include wild type NK-92^{®}, NK-92^{®}-CD16, NK-92^{®}-CD16-γ, NK-92^{®}-CD16-ξ, NK-92^{®}-CD16(F176V), NK-92^{®}MI, and NK-92^{®}CI. NK-92^{®} cells are known to persons of ordinary skill in the art, to whom such cells are readily available from NantKwest, Inc.

As used herein, the term "aNK^{™} cells" refers to the parental NK-92 cells.

As used herein, the term "haNK^{®} cells" refers to NK-92^{®} cells that have been engineered to express Fc receptor.

As used herein, the term "taNK^{®} cells" refers to NK-92^{®} cells that have been engineered to express a chimeric antigen receptor (CAR) with affinity for a cancer specific antigen, a cancer associated antigen, or a tumor specific antigen. In some embodiments, the tumor specific antigen is HER-2, e.g., human HER-2, and these NK-92^{®} cells are referred to as HER2.taNK^{®} cells in this disclosure.

The term "Fc receptor" refers to a protein found on the surface of certain cells (e.g., natural killer cells) that contribute to the protective functions of the immune cells by binding to part of an antibody known as the Fc region. Binding of the Fc region of an antibody to the Fc receptor (FcR) of a cell stimulates phagocytic or cytotoxic activity of a cell via antibody-mediated phagocytosis or antibody-dependent cell-mediated cytotoxicity (ADCC). FcRs are classified based on the type of antibody they recognize. For example, Fc-gamma receptors (FcyR) bind to the IgG class of antibodies. FcyRIII-A (also called CD16) is a low affinity Fc receptor bind to IgG antibodies and activate ADCC. FcyRIII-A are typically found on NK cells. NK-92^{®} cells do not express FcyRIII-A.

The term "chimeric antigen receptor" (CAR), as used herein, refers to an extracellular antigen-binding domain that is fused to an intracellular signaling domain. CARs can be expressed in T cells or NK cells to increase cytotoxicity. In general, the extracellular antigen-binding domain is a scFv that is specific for an antigen found on a cell of interest. A CAR-expressing NK-92^{®} cell is targeted to cells expressing certain antigens on the cell surface, based on the specificity of the scFv domain. The scFv domain can be engineered to recognize any antigen, including tumor-specific antigens.

The terms "polynucleotide", "nucleic acid" and "oligonucleotide" are used interchangeably and refer to a polymeric form of nucleotides of any length, either deoxyribonucleotides or ribonucleotides or analogs thereof. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, a polynucleotide encompasses both the double-stranded form and each of two complementary single-stranded forms known or predicted to make up the double-stranded form.

The term "expression" refers to the production of a gene product. The term "transient" when referred to expression means a polynucleotide is not incorporated into the genome of the cell.

The term "cytokine" or "cytokines" refers to the general class of biological molecules which effect cells of the immune system. Exemplary cytokines include, but are not limited to, interferons and interleukins (IL), in particular IL-2, IL-12, IL-15, IL-18 and IL-21. In preferred embodiments, the cytokine is IL-2.

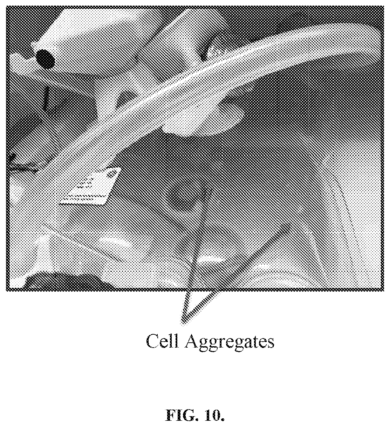

As used herein, the term "clumping" refers to the presence of cell clumps that are visible to the naked eye. A cell clump typically has a diameter of 1-15 cm, e.g., 2-10 cm, 2-8 cm, 1-3 cm, 2-6 cm, or 6-8 cm. Illustrative examples of visible cell clumps are shown in FIG. 10. In general, the degree of clumping of the cell culture increases as the volume of the cell culture increases, which typically occurs during expansion.

As used herein, the term "reduced clumping" or "reduced cell clumping" refers to the phenomenon that the number, the size, or both, of the cell clumps in the cell culture are reduced. For example, the number of cell clumps in a cell culture can be reduced by 1, 2, 3, 4, 5, 6, 7, 8, 9, 10, 15, 20, 25, 30, 35, 40, 45, 50, 55, 60, 65, 70, 75, 80, 85, 90, 95, or 99% as compared to a control cell culture using the provided methods.

The term "substantially free from clumping" refers to the culture condition that no cell clumping is visible to the naked eye.

As used herein, the term "cell aggregate" refers to an aggregate of five or more cells in a cell culture. Cell aggregates are typically not visible to naked eye but may be viewed with the aid of a device, such as a microscope. A reduction in cell aggregates refers to at least 40% reduction in the numbers of cell aggregretes in the cell culture as compared to a control cell culture. Optionally, the provided methods result in a reduction of at least 40, 45, 50, 55, 60, 65, 70, 75, 80, 85, 90, 95, 96, 97, 98, or 99% of the cell aggregates compared to a control cell culture.

As used herein, the term "fluocculant" refers to an aggregation of culture medium components in the absence of cells. A fluocculant is typically also visible to the naked eye and may typically have a diameter of 0.01-1cm, e.g., 0.02-0.8 cm, 0.05-0.5 cm, e.g., 0.1-0.5 cm.

As used herein, the term "reduced fluocculants" refers to the phenomenon that the number, the size, or both, of fluocculants in the medium are reduced. For example, the number of fluocculants in a culture medium can be reduced by 1, 2, 3, 4, 5, 6, 7, 8, 9, 10, 15, 20, 25, 30, 35, 40, 45, 50, 55, 60, 65, 70, 75, 80, 85, 90, 95, or 99% as compared to a control culture medium using the provided methods.

As used herein, the term "substantially the same cytotoxicity" refers to the that the two measurements from a cytotoxicity assay is no more than 15% different, no more than 10%, no more than 8%, or no more than 5% different from each other.

As used herein, the terms "cytotoxic" when used to describe the activity of effector cells such as NK cells, relates to killing of target cells by any of a variety of biological, biochemical, or biophysical mechanisms.

### CULTURE MEDIA

Provided herein is a culture medium comprising a non-ionic surfactant that can reduce or prevent clumping. The non-ionic surfactant can control shear forces in cell cultures and can also be used to reduce foaming in stirred cultures and reduce cell attachment to culture vessel.

Suitable non-ionic surfactants include Poloxamers. Poloxamers are non-ionic triblock copolymers composed of a central hydrophobic chain of polyoxypropylene flanked by two hydrophilic chains of polyoxyethylene. Poloxamers are also known by the trade names synperonics, pluronics, and kolliphor. Poloxamer 188 solutions are commercially available, for example, from Sigma-Aldrich or Thermo Fisher Scientific. Poloxamer 188 is referred to as Pluronic F-68 when obtained from Thermo Fisher Scientific. Poloxamer 188 is typically provided commercially at a concentration of 10%.

The non-ionic surfactant, e.g., Poloxamer 188, may be present in an amount of 0.025 to 0.9%, e.g., 0.025% to 0.06%, 0.04% to 0.06%, or 0.03 to 0.05%. Preferably, the non-ionic surfactant is present in 0.05%.

In order to produce biological products from NK-92^{®} cells in sufficient quantities, cell cultures need to be scaled up to a relatively large volume, for example, the volume of the cell culture may be at least 2 liters, at least 3 liters, at least 4 liters, or at least 5 liters. In some cases, NK-92^{®} cells can be grown in large-volume culture vessels that are suitable to be used in bioreactors. As stated before, large-volume cell cutures are often accompanied with significant increases in clumping in the cell culture, i.e., cell precipitation and aggregation. In the absence of Poloxamer 188, when a 2-Liter WAVE bag culture is scaled up in a 20-Liter WAVE bag, precipitation can form within 24 hours (as illustrated in Example 1, and Table 1). Adding Poloxamer 188 to cell cultures, including cultures having large volumes, surprisingly can significantly reduce clumping in the cell culture. Adding Poloxamer 188 to NK-92^{®} cell cultures does not adversely impact cell growth, cell viability, phenotype, cytotoxicity and ADCC activity. Accordingly, the disclosure also provides a method of culturing NK-92^{®} cells comprising culturing the NK-92^{®} cells in a culture medium comprising 0.025% to 0.9% of a non-ionic surfactant, wherein the NK-92^{®} cells have reduced clumping as compared to control NK-92^{®} cells that have been cultured in a control medium lacking the non-ionic surfactant, and the NK-92^{®} cells have substantially the same cytotoxicity as the control NK-92^{®} cells.

It has been also observed by inventors of this application that even in the absence of cells, cell culture media may form fluocculants that are visible to naked eye. These fluocculants typically have a diameter of 0.01-1cm, e.g., 0.02-0.8 cm, 0.05-0.5 cm, e.g., 0.1-0.5 cm and introducing 0.025% to 0.9% of Poloxamer 188 to the media can reduce the number of fluocculants in the medium.

### METHODS OF CULTURING NK-92^{®} CELLS

NK-92^{®} cells can be cultured in a number of growth media and some of which are commercially available, for example, human NK cell culture medium from 3H Biomedical (Uppsala, Sweden) or Prime XV medium from Irvine Scientific (Irvine, CA, USA). To minimize clumping, the culture may comprise Poloxamer 188. Optionally, the culture media also contain cytokines, human serum albumin, amino acids supplements, or combinations thereof. Suitable cytokines include, but are not limited to, IL-2. In one illustrative example, aNK cells may be cultured in growth medium comprising 0.05% Pluronic F-68 and 450 IU/mL of IL-2, with amino acids added as supplements. In another illustrative example, haNK^{®} cells may be cultured in growth medium comprising 0.05% Pluronic F-68. In yet another example, taNK^{®} cells can be cultured in a growth medium that comprises 0.05% Pluronic F-68, and 500 IU/mL of IL-2. In yet another example, t-haNK^{®} cells can be cultured in a growth medium that comprises 0.05-0.1% Pluronic F-68.

As described above, adding the non-ionic surfactant to the growth medium may reduce clumping. Optionally, adding the non-ionic surfactant to the grow medium can reduce clumping by at least 40%, at least 50%, at least 60%, at least 70%, at least 80%, at least 88%, at least 90%, or at least 95%.

Using the non-ionic surfactant, e.g., Poloxamer 188, in the cell culture can also reduce NK-92 cell aggregates to a significant degree as compared to a control cell culture that lacks the non-ionic surfactant. In some cases, it may reduce percentages of cell aggregates in the culture by at least 40%, at least 50%, at least 60%, at least 70%, at least 80%, at least 90%, or at least 95%. In some cases, NK-92 cells that grow in culture medium containing Poloxamer 188 may have less than 15%, less than 10%, less than 7%, less than 6% of cell aggregates after growing in a culture volume of at least 1 liter for a growth period. In some cases, the growth period is 1-7 days, for example, 2 days, 3 days, 5 days, or 7 days.

In order to expand NK-92^{®} cell culture, typically a vial is thaw and cultured in a standard cell culture vessel, e.g., a T75 flask, until the cells recover. The cells are then transferred to a vessel having a larger volume to expand the number of cells, e.g., a G-Rex Flask, which is typically less than 0.45 Liter. The expanded cells are then transferred to an even larger vessel, e.g., a WAVE cell culture bag. Transfer of cells can be performed wither using a pump or a gravity feed.

Optionally, NK-92^{®} cells so produced can be harvested using a continuous centrifuge that are aseptically attached to the culture vessel that is at the end of the expansion process. The cells can be collected and used for product formulation and various applications.

The cytotoxicity of the produced NK-92^{®} cells , the ability to target and kill aberrant cells, such as virally infected and tumorigenic cells, can be assessed by methods well known in the art, for example, a ⁵¹Cr release assay (Gong et al. (1994)) using the procedure described by Klingemann et al. (Cancer Immunol. Immunother. 33:395-397 (1991)). The percentage of specific cytotoxicity can be calculated based on the amount of released ⁵¹Cr. See Patent Pub. No. US20020068044.

Alternatively, the cytotoxicity of the produced NK-92^{®} cells can also be assessed using a calcein release assay. For example, the NK-92^{®} cells (referred to as the effector in the assay) can be mixed with the calcein loaded target cells (referred to as target in the assay) at certain ratios. After incubation for a period of time, the calcein release from the target cells can be assessed, e.g., by a fluorescence plate reader. The ratio of the effector and target used in the assay may vary, optionally the effector: target ratio may be 20:1, 15:1, 10:1, 8:1, or 5:1; preferably the effector: target ratio is 10:1. The NK-^{®}cells that have been grown in the media comprising Poloxamer 188, e.g., at concentrations between 0.025% to 0.9% maintain comparable cytotoxicity as the NK-92^{®} cells that have been grown in media that are identical but for the presence of the Poloxamer 188. The target cells can be any cells that express MHC molecules that can be recognized by the NK-92^{®} cells, for example, the K562 cells.

The antibody dependent cytotoxicity of the NK-92^{®} cells, e.g., haNK^{®} cells, can be assessed. Methods for measuring the ADCC of NK-92^{®} cells are similar to the methods of measuring direct cytotoxicity as described above except that an antibody that can recognize the target cell is added. The Fc receptor of the NK cells recognizes the cell-bound antibodies and triggers cytolytic reaction and killing the target cells. In one illustrative example, the haNK^{®} cells can be incubated with Rituxan (an antibody) and Ramos(target cells) and killing of the Ramos cells can be measured by the release of internal components of the target cells, e.g., ⁵¹Cr or calcein, as described above.

The NK-92^{®} cells that have been grown in a culture medium comprising 0.025%-0.9% of the non-ionic surfactant, e.g., Poloxamer 188, can demonstrate substantially the same cytotoxicity as the NK-92^{®} cells that have been grown in the a culture medium lacking the non-ionic surfactant.

The NK-92^{®} cells that have been grown in a culture medium comprising 0.025%-0.9% of the non-ionic surfactant, e.g., Poloxamer 188, typically have excellent viability, for example, a viability of at least 80%, at least 85%, at least 90%, or at least 95% . The viability of the NK-92^{®} cells can be determined using methods that are well known in the art, for example, a trypan blue-based staining method or an NC-200 cell counter.

### CELL CULTURE

This disclosure also provides a cell culture comprising NK-92^{®} cells and a culture medium comprising 0.025% to 0.9% of the non-ionic surfactant, wherein the NK-92^{®} cells have reduced clumping as compared to control culture comprising NK-92^{®} cells and a medium lacking the non-ionic surfactant.

Optionally, the cell culture may have reduced clumping as compared to a control cell culture that lacks the non-ionic surfactant and the reduction is by at least 40%, at least 50%, at least 60%, at least 70%, at least 80%, at least 88%, at least 90%, or at least 95%.

Optionally, the NK-92^{®} cells have been cultured in the culture medium for at least 3 days, at least 5 days, at least 7 days, or at least 10 days. Optionally, the cell culture has a volume of at least 2 liters, at least 5 liters, at least 10 liters, at least 15 liters, e.g., 25 liters. Optionally, the NK-92^{®} cell culture is substantially free from clumping.

Optionally, the non-ionic surfactant is Poloxamer 188. Optionally cell culture comprises 0.025% to 0.9%, e.g., 0.03% to 0.8%, or 0.04% to 0.6% of Poloxamer 188; preferably, the Poloxamer 188 is present at 0.05%.

### NK-92^{®} CELLS

The NK-92^{®} cells that can be cultured using the methods disclosed herein include aNK^{™} cells, haNK^{®} cells, taNK^{®} cells, and t-haNK^{®} cells which are further described below.

The NK-92^{®} cell line is a unique cell line that was discovered to proliferate in the presence of interleukin 2 (IL-2). Gong et al., Leukemia 8:652-658 (1994). These cells have high cytolytic activity against a variety of cancers. The NK-92^{®} cell line is a homogeneous cancerous NK cell population having broad anti-tumor cytotoxicity with predictable yield after expansion. Phase I clinical trials have confirmed its safety profile. NK-92^{®} was discovered in the blood of a subject suffering from a non-Hodgkins lymphoma and then immortalized *ex vivo.* NK-92^{®} cells are derived from NK cells, but lack the major inhibitory receptors that are displayed by normal NK cells, while retaining the majority of the activating receptors. NK-92^{®} cells do not, however, attack normal cells nor do they elicit an unacceptable immune rejection response in humans. Characterization of the NK-92^{®} cell line is disclosed in WO 1998/49268 and U.S. Patent Application Publication No. 2002-0068044.

The NK-92^{®} cell line is found to exhibit CD56^{bright}, CD2, CD7, CD11a, CD28, CD45, and CD54 surface markers. It furthermore does not display the CD1, CD3, CD4, CD5, CD8, CD10, CD14, CD16, CD19, CD20, CD23, and CD34 markers. Growth of NK-92^{®} cells in culture is dependent upon the presence of recombinant interleukin 2 (rIL-2), with a dose as low as 1 IU/mL being sufficient to maintain proliferation. IL-7 and IL-12 do not support long-term growth, nor do other cytokines tested, including IL-1α, IL-6, tumor necrosis factor α, interferon α, and interferon γ. NK-92^{®} has high cytotoxicity even at a low effector:target (E:T) ratio of 1:1. Gong, et al., *supra.* NK-92^{®} cells are deposited with the American Type Culture Collection (ATCC), designation CRL-2407.

Heretofore, studies on endogenous NK cells have indicated that IL-2 (1000 IU/mL) is critical for NK cell activation during shipment, but that the cells need not be maintained at 37 °C and 5% carbon dioxide. Koepsell, et al., Transfusion 53:398-403 (2013).

Modified NK-92^{®} cells are known and include, but are not limited to, those described in, e.g., U.S. Patent Nos. 7,618,817, 8,034,332, and 8,313,943, US Patent Application Publication No. 2013/0040386, such as wild type NK-92^{®}, NK-92^{®}-CD16, NK-92^{®}-CD16-γ, NK-92^{®}-CD16-ξ, NK-9^{®}2-CD16(F157V), NK-92^{®}mi and NK-92^{®}ci.

Although NK-92^{®} cells retain almost all of the activating receptors and cytolytic pathways associated with NK cells, they do not express CD16 on their cell surfaces. CD16 is an Fc receptor which recognizes and binds to the Fc portion of an antibody to activate NK cells for antibody-dependent cellular cytotoxicity (ADCC). Due to the absence of CD16 receptors, NK-92^{®} cells are unable to lyse target cells via the ADCC mechanism and, as such, cannot potentiate the anti-tumor effects of endogenous or exogenous antibodies (i.e., Rituximab and Herceptin).

Studies on endogenous NK cells have indicated that IL-2 (1000 IU/mL) is critical for NK cell activation during shipment, but that the cells need not be maintained at 37 °C and 5% carbon dioxide. Koepsell, et al., Transfusion 53:398-403 (2013). However, endogenous NK cells are significantly different from NK-92^{®} cells, in large part because of their distinct origins: NK-92^{®} is a cancer-derived cell line, whereas endogenous NK cells are harvested from a donor (or the patient) and processed for infusion into a patient. Endogenous NK cell preparations are heterogeneous cell populations, whereas NK-92^{®} cells are a homogeneous, clonal cell line. NK-92^{®} cells readily proliferate in culture while maintaining cytotoxicity, whereas endogenous NK cells do not. In addition, an endogenous heterogeneous population of NK cells does not aggregate at high density. Furthermore, endogenous NK cells express Fc receptors, including CD-16 receptors that are not expressed by NK-92^{®} cells.

### Fc receptors

Fc receptors bind to the Fc portion of antibodies. Several Fc receptors are known, and differ according to their preferred ligand, affinity, expression, and effect following binding to the antibody.

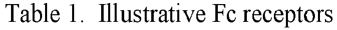

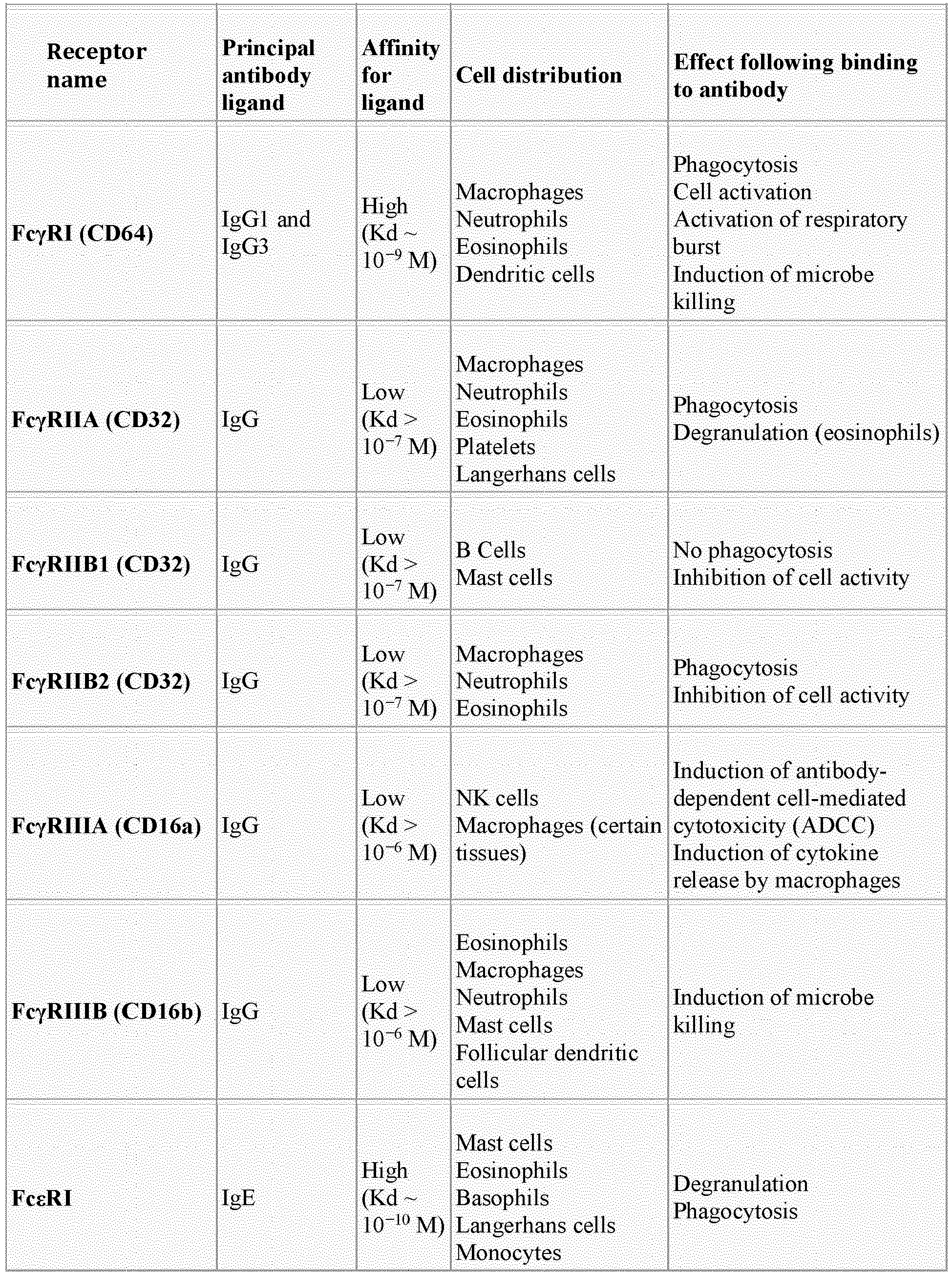

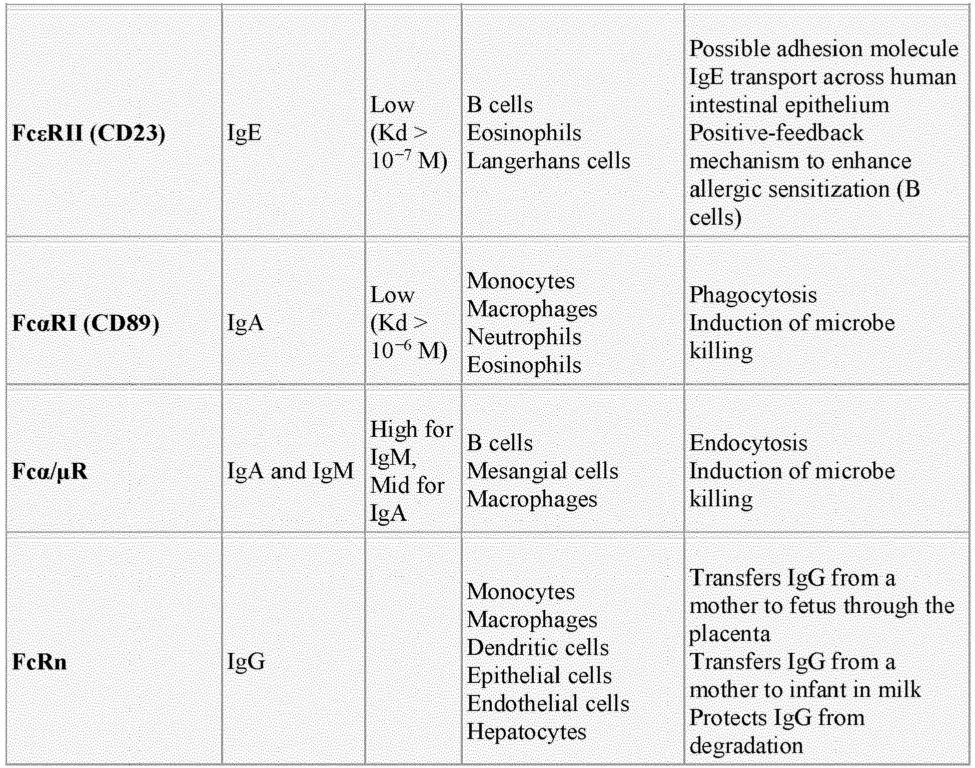

In some embodiments NK-92^{®} cells are modified to express an Fc receptor protein on the cell surface.

In some embodiments, the Fc receptor is human CD16. A representative amino acid sequence encoding CD16 is shown in SEQ ID NO:2. A representative polynucleotide sequence encoding CD16 is shown in SEQ ID NO:1. The complete sequences of CD16 can be found in the SwissProt database as entry P08637. In some embodiments, NK-92^{®} cells are modified by introducing a polynucleotide encoding a CD16 polypeptide has at least about 70% polynucleotide sequence identity with a polynucleotide sequence encoding a full-length, including signal peptide, naturally occurring CD16 that has a phenylalanine at position 176 of the full-length CD16. In some embodiments, a polynucleotide encoding a CD16 polypeptide has at least about 70% polynucleotide sequence identity with a polynucleotide sequence encoding a full-length, including the signal peptide, naturally occurring CD16 that has a valine at position 176.

Homologous polynucleotide sequences include those that encode polypeptide sequences coding for variants of CD16. In some embodiments, homologous CD16 polynucleotides may be about 150 to about 700, about 750, or about 800 polynucleotides in length, although CD16 variants having more than 700 to 800 polynucleotides are within the scope of the disclosure.

In other examples, cDNA sequences having polymorphisms that change the CD16 amino acid sequences are used to modify the NK-92^{®} cells, such as, for example, the allelic variations among individuals that exhibit genetic polymorphisms in CD16 genes. In other examples, CD16 genes from other species that have a polynucleotide sequence that differs from the sequence of human CD16 are used to modify NK-92^{®} cells.

In examples, variant polypeptides are made using methods known in the art such as oligonucleotide-mediated (site-directed) mutagenesis, alanine scanning, and PCR mutagenesis. Site direct mutagenesis (Carter, 1986; Zoller and Smith, 1987), cassette mutagenesis, restriction selection mutagenesis (Wells et al., 1985) or other known techniques can be performed on the cloned DNA to produce CD16 variants (Ausubel, 2002; Sambrook and Russell, 2001).

Conservative substitutions in the amino acid sequence of human CD16 polypeptide, whereby an amino acid of one class is replaced with another amino acid of the same class, fall within the scope of the disclosed CD16 variants as long as the substitution does not materially alter the activity of the polypeptide. Conservative substitutions are well known to one of skill in the art. Non-conservative substitutions that affect (1) the structure of the polypeptide backbone, such as a β-sheet or α-helical conformation, (2) the charge, (3) the hydrophobicity, or (4) the bulk of the side chain of the target site can modify CD16 polypeptide function or immunological identity. Non-conservative substitutions entail exchanging a member of one of these classes for another class. Substitutions may be introduced into conservative substitution sites or more preferably into non-conserved sites.

In some embodiments, CD16 polypeptide variants are at least 200 amino acids in length and have at least 70 % amino acid sequence identity, or at least 80%, or at least 90% identity to SEQ ID NO: 1 or SEQ ID NO:2. In some embodiments, CD16 polypeptide variants are at least 225 amino acid in length and have at least 70 % amino acid sequence identity, or at least 80%, or at least 90% identity to SEQ ID NO:1 or SEQ ID NO:2.

In some embodiments a nucleic acid encoding a CD16 polypeptide may encode a CD16 fusion protein. A CD16 fusion polypeptide includes any portion of CD16 or an entire CD16 fused with a non-CD16 polypeptide. In some embodiment, a fusion polypeptide may be created in which a heterologous polypeptide sequence is fused to the C-terminus of CD16 or is positioned internally in the CD16. Typically, up to about 30 % of the CD16 cytoplasmic domain may be replaced. Such modification can enhance expression or enhance cytotoxicity (e.g., ADCC responsiveness). In other examples, chimeric proteins, such as domains from other lymphocyte activating receptors, including but not limited to Ig-a, Ig-B, CD3-e, CD3-d, DAP-12 and DAP-10, replace a portion of the CD16 cytoplasmic domain.

Fusion genes can be synthesized by conventional techniques, including automated DNA synthesizers and PCR amplification using anchor primers that give rise to complementary overhangs between two consecutive gene fragments that can subsequently be annealed and reamplified to generate a chimeric gene sequence (Ausubel, 2002). Many vectors are commercially available that facilitate sub-cloning CD16 in-frame to a fusion moiety.

### Chimeric Antigen Receptor

As described herein, NK-92^{®} cells are further engineered to express a chimeric antigen receptor (CAR) on the cell surface. Optionally, the CAR is specific for a tumor- specific antigen. Tumor-specific antigens are described, by way of non-limiting example, in US 2013/0189268; WO 1999024566 A1; US 7098008 ; and WO 2000020460 A1. Tumor-specific antigens include, without limitation, NKG2D, CS1, GD2, CD138, EpCAM, EBNA3C, GPA7, CD244, CA-125, ETA, MAGE, CAGE, BAGE, HAGE, LAGE, PAGE, NY-SEO-1, GAGE, CEA, CD52, CD30, MUC5AC, c-Met, EGFR, FAB, WT-1, PSMA, NY-ESO1, AFP, CEA, CTAG1B, CD19 and CD33. Additional non-limiting tumor-associated antigens, and the malignancies associated therewith, can be found in Table 1.

**Table 1: Tumor-Specific Antigens and Associated Malignancies**

| **Target Antigen** | **Associated Malignancy** |
|---|---|
| α-Folate Receptor | Ovarian Cancer |
| CAIX | Renal Cell Carcinoma |
| CD19 | B-cell Malignancies |
| | Chronic lymphocytic leukemia (CLL) |
| | B-cell CLL (B-CLL) |
| | Acute lymphoblastic leukemia (ALL); ALL post Hematopoietic stem cell transplantation (HSCT) |
| | Lymphoma; Refractory Follicular Lymphoma; B-cell non-Hodgkin lymphoma (B-NHL) |
| | Leukemia |
| | B-cell Malignancies post-HSCT |
| | B-lineage Lymphoid Malignancies post umbilical cord blood transplantation (UCBT) |
| CD19/CD20 | Lymphoblastic Leukemia |
| CD20 | Lymphomas |
| | B-Cell Malignancies |
| | B-cell Lymphomas |
| | Mantle Cell Lymphoma |
| | Indolent B-NHL |
| | Leukemia |
| CD22 | B-cell Malignancies |
| CD30 | Lymphomas; Hodgkin Lymphoma |
| CD33 | AML |
| CD44v7/8 | Cervical Carcinoma |
| CD138 | Multiple Myeloma |
| CD244 | Neuroblastoma |
| CEA | Breast Cancer |
| | Colorectal Cancer |
| CS1 | Multiple Myeloma |
| EBNA3C | EBV Positive T-cells |
| EGP-2 | Multiple Malignancies |
| EGP-40 | Colorectal Cancer |
| EpCAM | Breast Carcinoma |
| Erb-B2 | Colorectal Cancer |
| | Breast Cancer and Others |
| | Prostate Cancer |
| Erb-B 2,3,4 | Breast Cancer and Others |
| FBP | Ovarian Cancer |
| Fetal Acetylcholine Receptor | Rhabdomyosarcoma |
| GD2 | Neuroblastoma |
| GD3 | Melanoma |
| GPA7 | Melanoma |
| Her2 | Breast Carcinoma |
| | Ovarian Cancer |
| | Tumors of Epithelial Origin |
| Her2/new | Medulloblastoma |
| | Lung Malignancy |
| | Advanced Osteosarcoma |
| | Glioblastoma |
| IL-13R-a2 | Glioma |
| | Glioblastoma |
| | Medulloblastoma |
| KDR | Tumor Neovasculature |
| k-light chain | B-cell Malignancies |
| | B-NHL, CLL |
| LeY | Carcinomas |
| | Epithelial Derived Tumors |
| L1 Cell Adhesion Molecule | Neuroblastoma |
| MAGE-A1 | Melanoma |
| Mesothelin | Various Tumors |
| MUC1 | Breast Cancer; Ovarian Cancer |
| NKG2D Ligands | Various Tumors |
| Oncofetal Antigen (h5T4) | Various Tumors |
| PSCA | Prostate Carcinoma |
| PSMA | Prostate/Tumor Vasculature |
| TAA Targeted by mAb IgE | Various Tumors |
| TAG-72 | Adenocarcinomas |
| VEGF-R2 | Tumor Neovasculature |

In some embodiments, the CAR targets CD19, CD33 or CSPG-4.

In examples, variant polypeptides are made using methods known in the art such as oligonucleotide-mediated (site-directed) mutagenesis, alanine scanning, and PCR mutagenesis. Site direct mutagenesis (Carter, 1986; Zoller and Smith, 1987), cassette mutagenesis, restriction selection mutagenesis (Wells et al., 1985) or other known techniques can be performed on the cloned DNA to produce CD16 variants (Ausubel, 2002; Sambrook and Russell, 2001).

Optionally, the CAR targets an antigen associated with a specific cancer type. Optionally, the cancer is selected from the group consisting of leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia), polycythemia vera, lymphomas (e.g., Hodgkin's disease and non-Hodgkin's disease), multiple myeloma, Waldenstrom's macroglobulinemia, heavy chain disease, solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma and retinoblastoma.

In some embodiments, a polynucleotide encoding a CAR is mutated to alter the amino acid sequence encoding for CAR without altering the function of the CAR. For example, polynucleotide substitutions leading to amino acid substitutions at "non-essential" amino acid residues can be made in the CARs disclosed above. CARs can be engineered as described, for example, in Patent Publication Nos. WO 2014039523; US 20140242701; US 20140274909; US 20130280285; and WO 2014099671. Optionally, the CAR is a CD19 CAR, a CD33 CAR or CSPG-4 CAR.

### Additional Modifications - Cytokines

The cytotoxicity of NK-92^{®} cells is dependent on the presence of cytokines (e.g., interleukin-2 (IL-2). The cost of using exogenously added IL-2 needed to maintain and expand NK-92^{®} cells in commercial scale culture is significant. The administration of IL-2 to human subjects in sufficient quantity to continue activation of NK92^{®} cells would cause adverse side effects.

In some embodiments, FcR-expressing NK-92^{®} cells are further modified to express at least one cytokine and a suicide gene. In specific embodiments, the at least one cytokine is IL-2, IL-12, IL-15, IL-18, IL-21 or a variant thereof. In preferred embodiments, the cytokine is human IL-2. A representative nucleic acid encoding IL-2 is shown in SEQ ID NO:3 and a representative polypeptide of IL-2 is shown in SEQ ID NO:4. In certain embodiments the IL-2 is a variant that is targeted to the endoplasmic reticulum.

In one embodiment, the IL-2 is expressed with a signal sequence that directs the IL-2 to the endoplasmic reticulum. Not to be bound by theory, but directing the IL-2 to the endoplasmic reticulum permits expression of IL-2 at levels sufficient for autocrine activation, but without releasing IL-2 extracellularly. See Konstantinidis et al "Targeting IL-2 to the endoplasmic reticulum confines autocrine growth stimulation to NK-92® cells" Exp Hematol. 2005 Feb;33(2):159-64. Continuous activation of the FcR-expressing NK-92^{®} cells can be prevented, *e.g.,* by the presence of the suicide gene.

### Additional Modifications - Suicide gene

The term "suicide gene" is one that allows for the negative selection of the cells. A suicide gene is used as a safety system, allowing the cells expressing the gene to be killed by introduction of a selective agent. This is desirable in case the recombinant gene causes a mutation leading to uncontrolled cell growth. A number of suicide gene systems have been identified, including the herpes simplex virus thymidine kinase (TK) gene, the cytosine deaminase gene, the varicella-zoster virus thymidine kinase gene, the nitroreductase gene, the *Escherichia coli* gpt gene, and the E. *coli* Deo gene (also see, for example, Yazawa K, Fisher W E, Brunicardi F C: Current progress in suicide gene therapy for cancer. World J. Surg. 2002 July; 26(7):783-9). As used herein, the suicide gene is active in NK-92^{®} cells. Typically, the suicide gene encodes for a protein that has no ill-effect on the cell but, in the presence of a specific compound, will kill the cell. Thus, the suicide gene is typically part of a system.

In one embodiment, the suicide gene is the thymidine kinase (TK) gene. The TK gene may be a wild-type or mutant TK gene *(e.g.,* tk30, tk75, sr39tk). Cells expressing the TK protein can be killed using ganciclovir.

In another embodiment, the suicide gene is Cytosine deaminase which is toxic to cells in the presence of 5-fluorocytosine. Garcia-Sanchez et al. "Cytosine deaminase adenoviral vector and 5-fluorocytosine selectively reduce breast cancer cells 1 million-fold when they contaminate hematopoietic cells: a potential purging method for autologous transplantation." Blood 1998 Jul 15;92(2):672-82.

In another embodiment, the suicide gene is cytochrome P450 which is toxic in the presence of ifosfamide, or cyclophosphamide. See e.g. Touati et al. "A suicide gene therapy combining the improvement of cyclophosphamide tumor cytotoxicity and the development of an anti-tumor immune response." Curr Gene Ther. 2014;14(3):236-46.

In another embodiment, the suicide gene is iCas9. Di Stasi, (2011) "Inducible apoptosis as a safety switch for adoptive cell therapy." N Engl J Med 365: 1673-1683. See also Morgan, "Live and Let Die: A New Suicide Gene Therapy Moves to the Clinic" Molecular Therapy (2012); 20: 11-13. The iCas9 protein induces apoptosis in the presence of a small molecule AP1903. AP1903 is biologically inert small molecule, that has been shown in clinical studies to be well tolerated, and has been used in the context of adoptive cell therapy.

In one embodiment, the modified NK-92^{®} cells are irradiated prior to administration to the patient. Irradiation of NK-92^{®} cells is described, for example, in U.S. Patent No. 8,034,332. In one embodiment, modified NK-92^{®} cells that have not been engineered to express a suicide gene are irradiated.

### Transgene expression

Transgenes (e.g., CD19 CAR and CD16) can be engineered into an expression vector by any mechanism known to those of skill in the art. Transgenes may be engineered into the same expression vector or a different expression vector. In preferred embodiments, the transgenes are engineered into the same vector.

In some embodiments, the vector allows incorporation of the transgene(s) into the genome of the cell. In some embodiments, the vectors have a positive selection marker. Positive selection markers include any genes that allow the cell to grow under conditions that would kill a cell not expressing the gene. Non-limiting examples include antibiotic resistance, e.g., geneticin (Neo gene from Tn5).

Any number of vectors can be used to express the Fc receptor and/or the CAR. In some embodiments, the vector is a plasmid. In one embodiment, the vector is a viral vector. Viral vectors include, but are not limited to, retroviral vectors, adenoviral vectors, adeno-associated viral vectors, herpes simplex viral vectors, pox viral vectors, and others.

Transgenes can be introduced into the NK-92^{®} cells using any transfection method known in the art, including, by way of non-limiting example, infection, electroporation, lipofection, nucleofection, or "gene-gun."

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutations of these compounds may not be explicitly disclosed, each is specifically contemplated and described herein. For example, if a method is disclosed and discussed and a number of modifications that can be made to a number of molecules including the method are discussed, each and every combination and permutation of the method, and the modifications that are possible are specifically contemplated unless specifically indicated to the contrary. Likewise, any subset or combination of these is also specifically contemplated and disclosed. This concept applies to all aspects of this disclosure including, but not limited to, steps in methods using the disclosed compositions. Thus, if there are a variety of additional steps that can be performed, it is understood that each of these additional steps can be performed with any specific method steps or combination of method steps of the disclosed methods, and that each such combination or subset of combinations is specifically contemplated and should be considered disclosed.

### EXAMPLES

The following examples are for illustrative purposes only and should not be interpreted as limitations. There are a variety of alternative techniques and procedures available to those of skill in the art which would similarly permit one to successfully perform the examples below.

### Example 1: Pluronic F-68 reduced clumping in haNK^{®} cell cultures

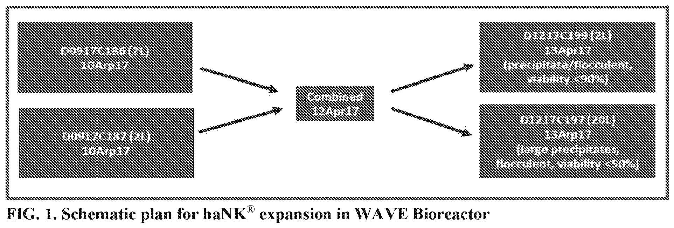

In one reference study, haNK^{®} cells from G-Rex flasks were inoculated into two separate 2-Liter WAVE bags (i.e., cell lot#D0917C186 and cell lot#D0917C187). Cells from both WAVE bags were combined to seed a 20-Liter WAVE bag (10L working volume) two days later. One day later, white precipitation and flocculants were observed in the 20-Liter WAVE bag D1217C197 (FIG. 10) and the viability of the cells were less than 50%. D1217C199 was simultaneously set up when the culture was expanded in the 20-Liter culture to serve as a backup culture; precipitation and flocculents were also observed and cell viability was less than 90%. See FIG. 1. This indicates the process is not scalable and the expansion was then terminated.

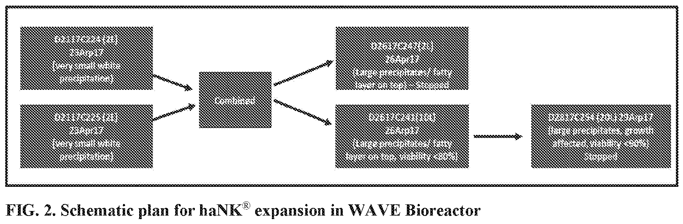

In another reference study, haNK^{®} cells from G-Rex Flasks were inoculated into two separate 2-Liter WAVE bags on day 0. Cells from both WAVEs were combined to seed a 10-Liter WAVE bag and a 2-Liter WAVE bag on day 3. Cells from 10-Liter WAVE bags were used to inoculate a 20-Liter WAVE bag on day 6. White precipitation and flocculants were observed in the 10-Liter WAVE bag. Cell growth was reduced and viability was less than 80%. Upon transfer to a 20-Liter bag, precipitate size grew bigger. The WAVE Culture was terminated. This also indicates that the process was not scalable. See FIG. 2.

As a working example, haNK^{®} cells from G-Rex Flasks were inoculated into two separate 2-Liter WAVE bags in the presence of 0.05% Pluronic F-68, purchased from Thermo Fisher Scientific as a 10% solution (Cat# 24040-032). Cells from both WAVE bags were combined to seed a 10-Liter WAVE bag (5L working volume) on day 0. Cells from 10-Liter WAVE bags were used to inoculate a two 20-Liter WAVE bag on day 4. Cells from both 20L WAVE bags were harvested by continuous centrifugation on separate days. All the WAVE cultures in this study comprised 0.05% pluronic F-68. The results show that no precipitates were observed in either of the WAVE bioreactors throughout the study.

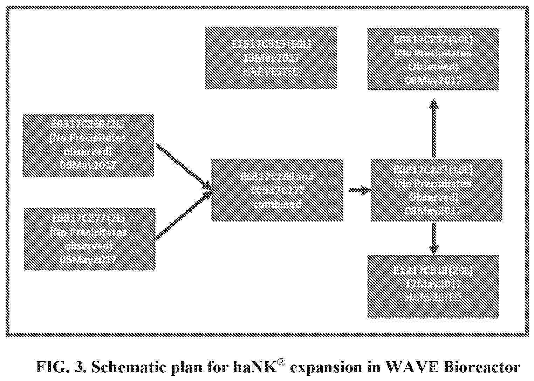

The results of these studies, as summarized in Table 1, indicate that 0.05% Pluronic F-68 was able to keep haNK^{®} culture from forming clumps and thus haNK^{®} cultures can be expanded by adding 0.05% Pluronic F-68 to the culture. See FIG. 3.

| **Experiment** | **Study Date and Number** | **Pluronic-F68 (0.05%v/v)** | **Precipitates in Culture** | **Process Scalable** |
|---|---|---|---|---|
| **haNK^{®} (Expansion #1)** | 04/10/2017, NKSTUDYPRT004 | No | Yes | No |
| **haNK^{®} (Expansion #2)** | 04/23/2017, NKSTUDYPRT004 | No | Yes | No |
| **haNK^{®} (Expansion #3)** | 04/10/2017, NKSTUDYPRT004 | Yes | No | Yes |

### Example 2: Pluronic F-68 reduced clumping in aNK^{™} cell cultures

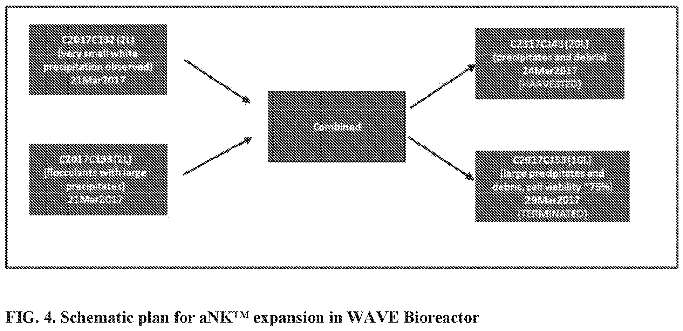

Similar experiments were performed with aNK^{™} cell culture. In one reference study, aNK^{™} cells from G-Rex Flasks were inoculated into two separate 2-Liter WAVE bags on day 0. Cells from both WAVEs were combined to seed a 20-Liter WAVE bag (10L working volume) and a 10-Liter WAVE bag (5L working volume). Cells from 20-Liter bag were harvested by continuous centrifugation on day 3 despite clumping in the cell culture. White precipitation and flocculants were observed in the 20-Liter WAVE bag three days later. The 10-Liter WAVE culture was stopped due to larger precipitates and low cell viability of about 75%. See FIG. 4

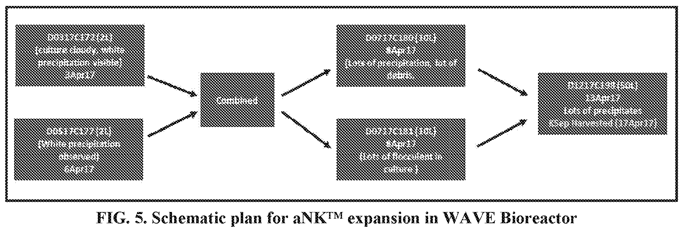

In another reference study, aNK^{™} cells from stirred-tank bioreactor were inoculated into two separate 2-Liter WAVE bags on day 0. Due to reduced viability <50%, culture in 20-Liter WAVE bag was terminated. Cells from both 2-Liter WAVEs were combined to seed a two separate 10-Liter WAVE bag on day 5. Cells from the 10-Liter WAVE bag were used for inoculation into a 50-Liter bag on day 10. Cells from 50-Liter bag were harvested by continuous centrifugation on day 14 despite clumping in the cell culture. White precipitation and flocculants were observed in the 20-Liter WAVE bag. Cell growth was reduced and hence expansion was stopped. This indicates that WAVE Culture had a lot of precipitation and the process was not scalable. See FIG. 5

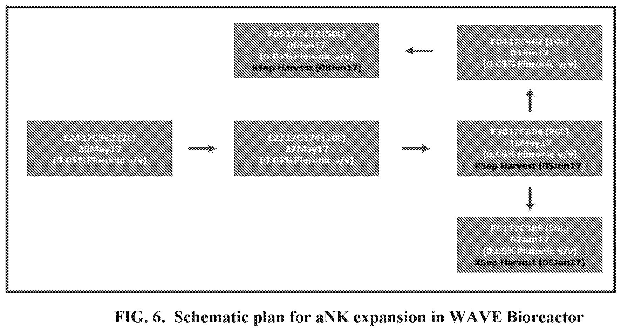

As a working example, aNK^{™} cells from G-Rex Flasks were inoculated into two separate 2-Liter WAVE bags in the presence of 0.05% Pluronic F-68 on day 0. Cells from both bioreactors were combined to seed a 10-Liter WAVE bag on day 5. Cells from 10-Liter bag were transferred to two separate 20-Liter WAVE bags. Cells from both bags were harvested using continuous centrifugation on day 14. No precipitation was observed in either of the WAVE bags. See FIG. 6. Cells grown in WAVE bioreactor Lot # F0517C412 showed potent activity against K562 target cells (see Table 3).

The results of these studies, as summarized in Table 2, indicate that haNK^{®} cultures can be expanded in the presence of 0.05% Pluronic F-68 without the occurrence of clumping in the culture.

| **Expenment,** | **Study Date and Number** | **Pluronic-F68 (0.05%v//v)** | **Precipitates in Culture** | **Process Scalable** |
|---|---|---|---|---|
| **aNK (Expansion #1)** | 03/21/2017, NKSTUDYPRT003 | No | Yes | No |
| **aNK (Expansion #2)** | 04/03/2017, NKSTUDYPRT003 | No | Yes | No |
| **aNK (Expansion #3)** | 05/25/2017, NKSTUDYTP001 | Yes | No | Yes |

### Example 3: Cytotoxicity of NK-92^{®} cells that have been cultured in Pluronic F-68-containing media

A sample of haNK cells that had been cultured in 20-Liter WAVE bags in the presence of Pluronic F-68 (Lot# E1217C313) were tested for antibody dependent cytotoxicity (ADCC). A reference sample from the flask before the expansion, which had not been treated with Pluronic F-68, was also tested simultaneously. The sample or the reference sample was mixed with calcein-loaded target cells, Ramos cells, at the effector: target ratio of 10:1 in the presence of Rituxan antibody. Calcein release was measured by fluorescence plate reader post 3 hours of incubation. Cytotoxicity was expressed as percentage of calcein release. Each sample was tested in triplicate and the results are shown in Table 3,

Similarly, a sample of aNK^{™} cells that had been cultured in 20-Liter WAVE bags in the presence of Pluronic F-68 (Lot# F0517C412) was tested for cytotoxicity. A reference sample from the flask before the expansion, which had not been treated with Pluronic F-68, was also tested simultaneously. The sample or the reference sample was mixed with calcein-loaded target cells, K562 cells, at the effector: target ratio of 10:1. Calcein release was assessed by a fluorescence plate reader post 3 hours of incubation. Cytotoxicity was expressed as percentage of calcein release. Each sample was tested in triplicate and the results are shown in Table 3.

| **Table 3. Cytotoxicity of the NK-92^{®} cells treated with Pluronic F-68** | | | |
|---|---|---|---|
| **Experiment** | **Study Date and Number** | **Culture Vessel/Stage** | **% Cytotoxicity** |
| **haNK WAVE E1217C313** | 05/17/2017, NKSTUDYPRT004 | Test Sample | 107 ± 4 |
| | | Flask Reference | 115 ± 2 |
| **aNK^{™} WAVE F0517C412** | 06/08/2017, NKSTUDYTP002 | Test Sample | 103 ± 1 |
| | | Flask Reference | 97 ± 5 |

The results, as shown in Table 3, indicate that the cytotoxicity of the cells treated with Pluronic F-68 is substantially the same as the cytotoxicity of those not so treated, suggesting adding Pluronic F-68 to growth media does not adversely affect the cytotoxicity of NK-92^{®} cells.

### Example 4: Identify the minimum effective concentration of Pluronic F-68 required to prevent clumping in NK-92^{®} cell culture

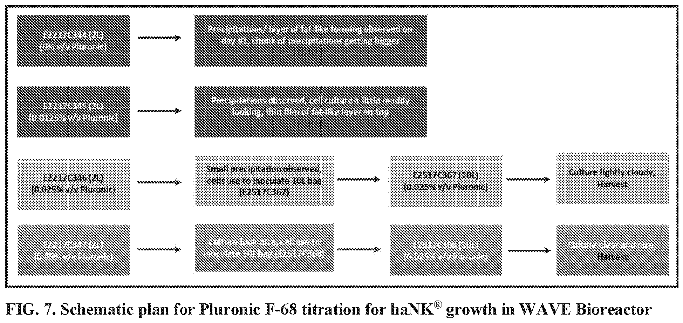

This example describes studies conducted to identify the minimum effective concentration of Pluronic F-68 required to prevent clumping. Pluronic F-68 was added to the haNK cell culture in WAVE bioreactor at concentrations of 0%, 0.0125%, 0.025% and 0.05%, respectively. Clumping was observed in culture with no or 0.125% Pluronic F-68. Clumping was still also in culture with 0.025% Pluronic F-68 albeit at a reduced amount. Notably, Clumping and cloudiness were completely prevented by addition of 0.05% Pluronic F-68. See FIG. 7. Due to cloudy appearance, two of the WAVE cultures (0% and 0.0125% Pluronic F-68) were terminated while the other two cultures were expanded in separate 10 L bags for another 5 days. Cells were analyzed using an NC-200 cell counter and percentages of cell aggregates are shown in Table 4.

| | **Table 4. Cell aggregates in growth media having various concentrations of Pluronic F-68** | | |
|---|---|---|---|
| **Pluronic concentration** | Cell aggregates after 3 days in 1 L WAVE bag | Reduction in cell aggregates | Cell aggregates after 5 days in 10 L WAVE bag |
| 0% | 9% | - | NA |
| 0.0125% | 6% | 33% | NA |
| 0.025% | 5% | 44% | 7% |
| 0.05% | 1% | 89% | 2% |

### Example 5. Evaluate the effect of Pluronic F-68 on NK-92^{®} cell viability using an NC-200 cell counter

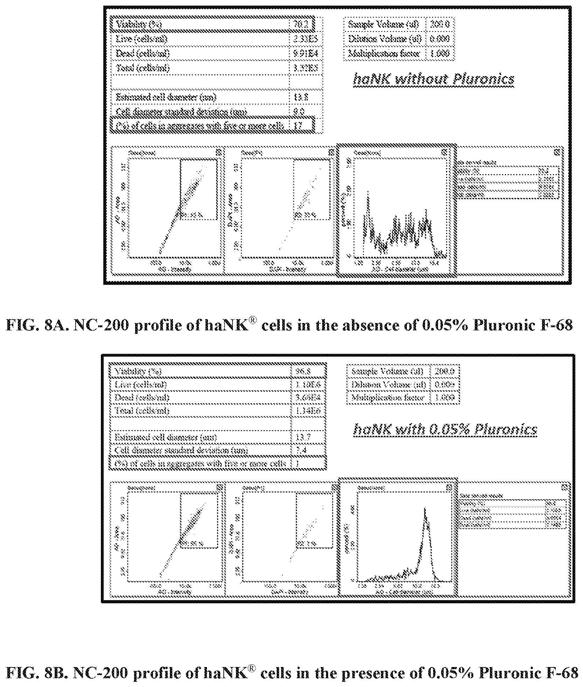

haNK^{®} cells that had been expanded in the absence or presence of Pluronic F-68 in a WAVE bioreactor were evaluated for their health and viability using a NC-200 cell counter. As shown in FIG. 8A, multiple peaks appeared in an Acridine Orange plot from the culture having no Pluronic F-68, indicating the culture was unhealthy. Cell viability was 70.2% and cells that were in aggregates with five or more cells account for 17% of the total cells in the sample. In contrast, only a single peak was observed in the cultures containing 0.05% Pluronic F-68, indicating that the cells were healthy. In addition, cell viability increased to 94.9% and the cells were substantially free from clumping, as indicated by that the percentage of cells in aggregates with five or more cells was only 2% (FIG. 8B). Adding Pluronic F-68 reduced clumping by 88%.

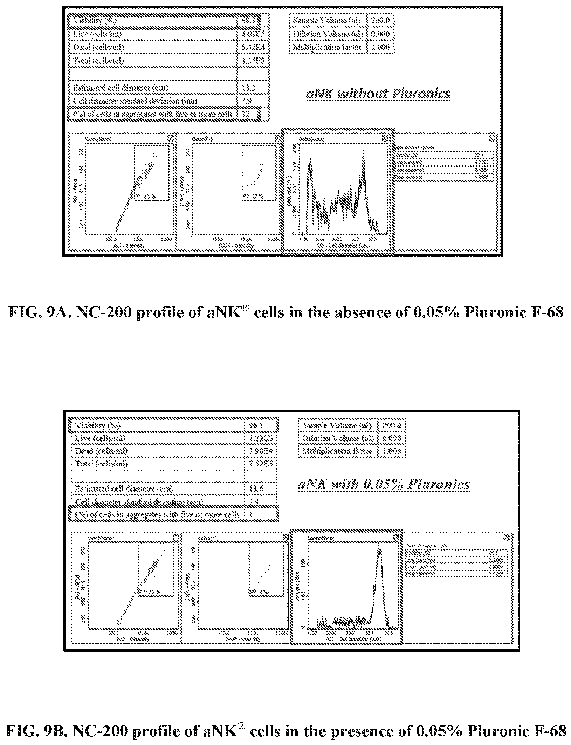

Similar studies were performed on aNK^{™} cells that were expanded in the absence or presence of Pluronic F-68 in a WAVE bioreactor using an NC-200 cell counter. As with the haNK^{®} cells, multiple peaks were shown in an Acridine Orange plot from the culture having no Pluronic F-68, indicating the culture was unhealthy. Cell viability was 88.1% and 32% of cells were in aggregates with five or more cells. See FIG. 9A. In contrast, only a single peak was observed in the cultures containing 0.05% Pluronic F-68, indicating that the cells were healthy. In addition, cell viability improved to 96.8% and percentage of cells aggregates was reduced to mere 3% (FIG. 9B) - a reduction of 90%.

### Informal Sequence Listing

**SEQ ID NO: 1 High Affinity Variant Immunoglobulin Gamma Fc Region Receptor III-A nucleic acid sequence (full length form).** 
ATGTGGCA GCTGCTGCTG CCTACAGCTC TCCTGCTGCT GGTGTCCGCC
GGCATGAGAA CCGAGGATCT GCCTAAGGCC GTGGTGTTCC TGGAACCCCA
GTGGTACAGA GTGCTGGAAA AGGACAGCGT GACCCTGAAG TGCCAGGGCG
CCTACAGCCC CGAGGACAAT AGCACCCAGT GGTTCCACAA CGAGAGCCTG
ATCAGCAGCC AGGCCAGCAG CTACTTCATCGACGCCGCCA CCGTGGACGA
CAGCGGCGAG TATAGATGCC AGACCAACCT GAGCACCCTGAGCGACCCCG
TGCAGCTGGA AGTGCACATC GGATGGCTGC TGCTGCAGGC
CCCCAGATGGGTGTTCAAAG AAGAGGACCC CATCCACCTG AGATGCCACT
CTTGGAAGAA CACCGCCCTGCACAAAGTGA CCTACCTGCA GAACGGCAAG
GGCAGAAAGT ACTTCCACCA CAACAGCGAC TTCTACATCC CCAAGGCCAC
CCTGAAGGAC TCCGGCTCCT ACTTCTGCAG AGGCCTCGTGGGCAGCAAGA
ACGTGTCCAG CGAGACAGTG AACATCACCA TCACCCAGGG
CCTGGCCGTGTCTACCATCA GCAGCTTTTT CCCACCCGGC TACCAGGTGT
CCTTCTGCCT CGTGATGGTGCTGCTGTTCG CCGTGGACAC CGGCCTGTAC
TTCAGCGTGA AAACAAACAT CAGAAGCAGCACCCGGGACT GGAAGGACCA
CAAGTTCAAG TGGCGGAAGG ACCCCCAGGA CAAGTGA
**SEQ ID NO: 2 High Affinity Variant Immunoglobulin Gamma Fc Region Receptor III-A amino acid sequence (full length form). The Val at position 176 is underlined.** 
Met Trp Gln Leu Leu Leu Pro Thr Ala Leu Leu Leu Leu Val Ser Ala Gly Met Arg Thr Glu Asp
Leu Pro Lys Ala Val Val Phe Leu Glu Pro Gln Trp Tyr Arg Val Leu Glu Lys Asp Ser Val Thr
Leu Lys Cys Gln Gly Ala Tyr Ser Pro Glu Asp Asn Ser Thr Gln Trp Phe His Asn Glu Ser Leu
Ile Ser Ser Gln Ala Ser Ser Tyr Phe Ile Asp Ala Ala Thr Val Asp Asp Ser Gly Glu Tyr Arg Cys
Gln Thr Asn Leu Ser Thr Leu Ser Asp Pro Val Gln Leu Glu Val His Ile Gly Trp Leu Leu Leu
Gln Ala Pro Arg Trp Val Phe Lys Glu Glu Asp Pro Ile His Leu Arg Cys His Ser Trp Lys Asn
Thr Ala Leu His Lys Val Thr Tyr Leu Gln Asn Gly Lys Gly Arg Lys Tyr Phe His His Asn Ser
 
Asp Phe Tyr Ile Pro Lys Ala Thr Leu Lys Asp Ser Gly Ser Tyr Phe Cys Arg Gly Leu Val Gly
Ser Lys Asn Val Ser Ser Glu Thr Val Asn Ile Thr Ile Thr Gln Gly Leu Ala Val Ser Thr Ile Ser
Ser Phe Phe Pro Pro Gly Tyr Gln Val Ser Phe Cys Leu Val Met Val Leu Leu Phe Ala Val Asp
Thr Gly Leu Tyr Phe Ser Val Lys Thr Asn Ile Arg Ser Ser Thr Arg Asp Trp Lys Asp His Lys
Phe Lys Trp Arg Lys Asp Pro Gln Asp Lys
SEQ ID NO: 3 ER IL-2 nucleic acid sequence 
ATGTACCGGATG CAGCTGCTGA GCTGTATCGC CCTGTCTCTG GCCCTCGTGA
CCAACAGCGC CCCTACCAGC AGCAGCACCA AGAAAACCCA GCTGCAGCTG
GAACATCTGC TGCTGGACCTGCAGATGATC CTGAACGGCA TCAACAACTA
CAAGAACCCC AAGCTGACCC GGATGCTGACCTTCAAGTTC TACATGCCCA
AGAAGGCCAC CGAACTGAAA CATCTGCAGT GCCTGGAAGAGGAACTGAAG
CCCCTGGAAG AAGTGCTGAA CCTGGCCCAG AGCAAGAACT
TCCACCTGAGGCCCAGGGAC CTGATCAGCA ACATCAACGT GATCGTGCTG
GAACTGAAAG GCAGCGAGACAACCTTCATG TGCGAGTACG CCGACGAGAC
AGCTACCATC GTGGAATTTC TGAACCGGTGGATCACCTTC TGCCAGAGCA
TCATCAGCAC CCTGACCGGC TCCGAGAAGG ACGAGCTGTGA
SEQ ID NO: 4 ER IL-2 (ER retention signal is underlined) amino acid sequence

Met Tyr Arg Met Gln Leu Leu Ser Cys Ile Ala Leu Ser Leu Ala Leu Val Thr Asn Ser Ala Pro
Thr Ser Ser Ser Thr Lys Lys Thr Gln Leu Gln Leu Glu His Leu Leu Leu Asp Leu Gln Met Ile
Leu Asn Gly Ile Asn Asn Tyr Lys Asn Pro Lys Leu Thr Arg Met Leu Thr Phe Lys Phe Tyr Met
Pro Lys Lys Ala Thr Glu Leu Lys His Leu Gln Cys Leu Glu Glu Glu Leu Lys Pro Leu Glu Glu
Val Leu Asn Leu Ala Gln Ser Lys Asn Phe His Leu Arg Pro Arg Asp Leu Ile Ser Asn Ile Asn
Val Ile Val Leu Glu Leu Lys Gly Ser Glu Thr Thr Phe Met Cys Glu Tyr Ala Asp Glu Thr Ala
Thr Ile Val Glu Phe Leu Asn Arg Trp Ile Thr Phe Cys Gln Ser Ile Ile Ser Thr Leu Thr Gly Ser
Glu Lys Asp Glu Leu

## Claims

1. A method of culturing NK-92 cells comprising culturing the NK-92 cells in a culture medium comprising 0.025% to 0.9% of a non-ionic surfactant, wherein the NK-92 cell culture has reduced clumping as compared to control NK-92 cells that have been cultured in a control medium lacking the non-ionic surfactant.

2. The method of claim 1, wherein the NK-92 cells maintained the substantially the same cytotoxicity as the control NK-92 cells.

3. The method of claim 1, wherein the cell culture is substantially free from clumping.

4. The method of claim 1, wherein the cells are cultured in at least 2 liters of culture medium.

5. The method of claim 1, wherein the non-ionic surfactant is Poloxamer 188.

6. The method of claim 5, wherein the culture medium comprises from 0.025% to 0.06% of Poloxamer 188.

7. The method of claim 5, wherein the culture medium comprises 0.05% of the Poloxamer 188.

8. The method of claim 1, wherein the NK-92 cell culture has reduced cell aggregates as compared to a control cell culture.

9. The method of claim 8, wherein reduction of the percentage of cell aggregates is at least 40%.

10. The method of claim 8, wherein the NK-92 cell culture has less than 6% cell aggregates after 3 days of culturing.

11. The method of claim 1, wherein the NK-92 cells have a viability of at least 80%.

12. The method of claim 1, wherein the NK-92 cells comprise a cytokine, Fc Receptor, chimeric antigen receptor or a combination thereof.

13. A method of reducing fluocculants in the medium of a culture of NK92 cells the method comprising adding to the culture medium 0.025% to 0.9% of a non-ionic surfactant, wherein the culture medium has reduced fluocculants as compared to control culture lacking the non-ionic surfactant.

14. A cell culture comprising NK-92 cells and a culture medium comprising 0.025% to 0.9% of the non-ionic surfactant, wherein the cell culture has reduced clumping as compared to control culture comprising NK-92 cells and a medium lacking the non-ionic surfactant.

15. The cell culture of claim 14, wherein:
the NK-92 cells have been cultured for at least 3 days; or
the cell culture is substantially free from clumping; or
the NK-92 cells maintained the substantially the same cytotoxicity as the NK-92 cells in the control culture; or
the non-ionic surfactant is Poloxamer 188; or
the cell culture comprises 0.025% to 0.06% of the Poloxamer 188; or
the cell culture comprises 0.05% of the Poloxamer 188; or
the NK-92 cells comprise a cytokine, Fc Receptor, chimeric antigen receptor, or
a combination thereof; or
the cell culture has a volume of at least 2 liters or at least 10 liters.

## Patentansprüche

1. Verfahren zum Kultivieren von NK-92-Zellen, umfassend das Kultivieren der NK-92-Zellen in einem Kulturmedium, das 0,025 % bis 0,9 % eines nichtionischen Tensids umfasst, wobei die NK-92-Zellkultur reduzierte Verklumpung verglichen mit Kontroll-NK-92-Zellen aufweist, die in einem Kontrollmedium kultiviert worden sind, dem das nichtionische Tensid fehlt.

2. Verfahren nach Anspruch 1, wobei die NK-92-Zellen die im Wesentlichen die gleiche Zytotoxizität wie die Kontroll-NK-92-Zellen beibehielten.

3. Verfahren nach Anspruch 1, wobei die Zellkultur im Wesentlichen frei von Verklumpung ist.

4. Verfahren nach Anspruch 1, wobei die Zellen in zumindest 2 Litern Kulturmedium kultiviert werden.

5. Verfahren nach Anspruch 1, wobei das nichtionische Tensid Poloxamer 188 ist.

6. Verfahren nach Anspruch 5, wobei das Kulturmedium 0,025 % bis 0,06 % Poloxamer 188 umfasst.

7. Verfahren nach Anspruch 5, wobei das Kulturmedium 0,05 % des Poloxamer 188 umfasst.

8. Verfahren nach Anspruch 1, wobei die NK-92-Zellkultur reduzierte Zellaggregate verglichen mit einer Kontrollzellkultur aufweist.

9. Verfahren nach Anspruch 8, wobei Reduzierung des Prozentsatzes an Zellaggregaten zumindest 40 % ist.

10. Verfahren nach Anspruch 8, wobei die NK-92-Zellkultur weniger als 6 % Zellaggregate nach 3 Tagen Kultivierung aufweist.

11. Verfahren nach Anspruch 1, wobei die NK-92-Zellen eine Lebensfähigkeit von zumindest 80 % aufweisen.

12. Verfahren nach Anspruch 1, wobei die NK-92-Zellen ein Zytokin, einen Fc-Rezeptor, einen chimären Antigenrezeptor oder eine Kombination davon umfassen.

13. Verfahren zum Reduzieren von Fluokkulanten in dem Medium einer Kultur von NK92-Zellen, wobei das Verfahren das Hinzufügen von 0,025 % bis 0,9 % eines nichtionischen Tensids zu dem Kulturmedium umfasst, wobei das Kulturmedium reduzierte Fluokkulanten verglichen mit Kontrollkultur aufweist, der das nichtionische Tensid fehlt.

14. Zellkultur, umfassend NK-92-Zellen und ein Kulturmedium, das 0,025 % bis 0,9 % des nichtionischen Tensids umfasst, wobei die Zellkultur reduzierte Verklumpung verglichen mit Kontrollkultur aufweist, die NK-92-Zellen und ein Medium umfasst, dem das nichtionische Tensid fehlt.

15. Zellkultur nach Anspruch 14, wobei:
die NK-92-Zellen für zumindest 3 Tage kultiviert worden sind; oder
die Zellkultur im Wesentlichen frei von Verklumpung ist; oder
die NK-92-Zellen die im Wesentlichen die gleiche Zytotoxizität wie die NK-92-Zellen in der Kontrollkultur beibehielten; oder
das nichtionische Tensid Poloxamer 188 ist; oder
die Zellkultur 0,025 % bis 0,06 % des Poloxamer 188 umfasst; oder
die Zellkultur 0,05 % des Poloxamer 188 umfasst; oder
die NK-92-Zellen ein Zytokin, einen Fc-Rezeptor, einen chimären Antigenrezeptor oder eine Kombination davon umfassen; oder
die Zellkultur ein Volumen von zumindest 2 Litern oder zumindest 10 Litern aufweist.

## Revendications

1. Procédé de culture de cellules NK-92 comprenant la culture des cellules NK-92 dans un milieu de culture comprenant 0,025 % à 0,9 % d'un tensioactif non ionique, ladite culture de cellules NK-92 présentant une agglutination réduite par rapport aux cellules NK-92 témoins qui ont été cultivées dans un milieu témoin dépourvu du tensioactif non ionique.

2. Procédé selon la revendication 1, lesdites cellules NK-92 ayant conservé la sensiblement la même cytotoxicité que les cellules NK-92 témoins.

3. Procédé selon la revendication 1, ladite culture de cellules étant sensiblement exempte d'agglutination.

4. Procédé selon la revendication 1, lesdites cellules étant cultivées dans au moins 2 litres de milieu de culture.

5. Procédé selon la revendication 1, ledit tensioactif non ionique étant le poloxamère 188.

6. Procédé selon la revendication 5, ledit milieu de culture comprenant de 0,025 % à 0,06 % de poloxamère 188.

7. Procédé selon la revendication 5, ledit milieu de culture comprenant 0,05 % de poloxamère 188.

8. Procédé selon la revendication 1, ladite culture de cellules NK-92 comportant des agrégats cellulaires réduits par rapport à une culture de cellules témoin.

9. Procédé selon la revendication 8, ladite réduction du pourcentage d'agrégats cellulaires étant d'au moins 40 %.

10. Procédé selon la revendication 8, ladite culture de cellules NK-92 contenant moins de 6 % d'agrégats cellulaires après 3 jours de culture.

11. Procédé selon la revendication 1, lesdites cellules NK-92 présentant une viabilité d'au moins 80 %.

12. Procédé selon la revendication 1, lesdites cellules NK-92 comprenant une cytokine, un récepteur Fc, un récepteur d'antigène chimère ou une combinaison de ceux-ci.

13. Procédé de réduction des agents de fluoculation dans le milieu d'une culture de cellules NK92, le procédé comprenant l'ajout au milieu de culture de 0,025 % à 0,9 % d'un tensioactif non ionique, ledit milieu de culture comportant des agents de fluoculation réduits par rapport à une culture témoin dépourvue du tensioactif non ionique.

14. Culture de cellules comprenant des cellules NK-92 et un milieu de culture comprenant 0,025 % à 0,9 % du tensioactif non ionique, ladite culture de cellules présentant une agglutination réduite par rapport à une culture témoin comprenant des cellules NK-92 et un milieu dépourvu du tensioactif non ionique.

15. Culture de cellules selon la revendication 14 :
lesdites cellules NK-92 ayant été cultivées pendant au moins 3 jours ; ou
ladite culture de cellules étant sensiblement exempte d'agglutination ; ou
lesdites cellules NK-92 ayant conservé sensiblement la même cytotoxicité que les cellules NK-92 dans la culture témoin ; ou
ledit tensioactif non ionique étant le poloxamère 188 ; ou
ladite culture de cellules comprenant 0,025 % à 0,06 % du poloxamère 188; ou
ladite culture de cellules comprenant 0,05 % du poloxamère 188; ou
lesdites cellules NK-92 comprenant une cytokine, un récepteur Fc, un récepteur d'antigène chimère ou une combinaison de ceux-ci ; ou
ladite culture de cellules présentant un volume d'au moins 2 litres ou d'au moins 10 litres.
